# EUROPEAN PATENT APPLICATION

(11) **EP 0 567 678 A1**
(43) Date of publication of application: **03.11.1993**
(21) Application number: 92107343.3
(22) Date of filing: 29.04.1992
(51) Int. Cl.: A61B 19/00, A61L 2/18, B65D 83/14

(54) **Fluid dispensing device for disinfection of the hands**

(71) Applicant: Mongkol, Jesadanont, Jatujak, Bangkok 10900 (TH)
(72) Inventor: Mongkol, Jesadanont, Jatujak, Bangkok 10900 (TH)
(74) Representative: Prechtel, Jörg, Dipl.-Phys. Dr.

(57) **Abstract**

The technical problem to be solved was that of providing a device which could spray fluids and readily irrigate in a fully automated manner the entire surface of the hands. The solution to the problem is provided by a device having a housing comprises of two chambers. In the first chamber (A), the power supplies (2,6), a control circuit (3), a counter circuit (5) and a solid state relay (4) are installed. In the second chamber (B), a spray bottle (9) is installed in an upside-down manner with an electromagnet (7) and an iron frame atop. At the bottom of the device, an infrared sensor (1) is arranged which responds to the presence of hands located in its direct proximity underneath. Upon activation, the fluid disinfectant is sprayed evenly on the hands to be irrigated to ensure efficient dermatological treatment where using of any hand drier devices is unnecessary.

## Description

### BACKGROUND OF THE INVENTION

The invention relates to a fluid-spraying device for the dermatological treatment of the hands by dispensing disinfectant.

Previously, some apparata have been invented which can sprinkle or nebulize liquids of certain description over different parts of the human body, such as the face or limbs. The inventions in the past disclose devices which can dispense various liquids for medical or disinfection purposes by any one involved on account of his/her activity in contacts with a large number of people or special environments, and is to avoid contagion or becoming a contagion carrier.

Such prior apparata are only of moderate success even where intentionally designed for this particular purpose. Disadvantages are experienced with such apparata which affect adversely their usefulness.

It should be emphasized that the effectiveness of a treatment can be satisfactory only where the fluids are sprayed. This ensures deep penetration of the fluid droplets into the skin and optimized the hygienic condition because no liquid build-ups are produced and no devices are required for collecting and cleaning of the leftover fluids. Spraying makes unnecessary any hand drier devices, which devices are most of the time contaminated. In addition, the volatile disinfectants are to be used here. Rubbing hands thoroughly together would allow proper spreading such that irrigation would be most effective for both the hand palms and the back of the hands. Both hands would be completely disinfected with no need of contact any other surfaces thereafter.

With prior apparata, the above-mentioned problems are caused by the fact that the liquids are often just sprinkled over and not sprayed, and by that the irrigation is more likely to concern the back of the hands than the palms, where the fact is it is the hand palms that mostly require irrigation.

It should be further noted that an added disadvantages of some prior apparata comes from that they have in general a significant operating inertia, which needs an ample time interval before the next cycle can begin. This shortcoming imposes limitations on the practical use of known apparata in medical ambulatories and wherever the continued utilization of the apparata by a large number of persons is imperative.

Prior apparata, moreover, are relatively complex, expensive and bulky, and accordingly, basically unsuit ed to be widely used by the health professionals thereof which would be highly desirable for the hygienic reasons.

### SUMMARY OF THE INVENTION

The object and advantages of this invention are achieved through providing a fully automated spraying device for the dermatological treatment of the hands, comprising a housing having two chambers, one containing two power supplies, a control circuit, a counter circuit and a solid state relay. The other chamber contains a spray bottle filled with said disinfectant, an electromagnet, an iron frame and an infrared light sensor. Upon introduction of the hands underneath an infrared light sensor in its direct proximity, an electromagnet operative to punch down a spray bottle, a spray nozzle operative to dispense said volatile fluid disinfectant into the hand palms. The time interval between successive cycles is negligible such that it can be used continuously and a counter circuit make it possible to notify when the said fluid in said spray bottle is nearly used up by starting a warning sound. On no part of the apparatus is the disinfectant leftover such that cleaning is not necessary.

### BRIEF DESCRIPTION OF THE DRAWINGS

FIG. 1 shows a front sectional view of the device.
FIG. 2 is a sectional view of the device as taken along the line 1-1 of FIG. 1 showing the position of the infrared light sensor
FIG. 3 is a further sectional view of this device, taken along the line 2-2 of FIG. 1.
FIG. 4 is the sectional views of the spray nozzle
FIG. 5 shows the sectional view of a spray bottle operative in a usual manner.
FIG. 6 shows the position of the hands introduced under the device, with the disinfectant being sprayed onto.

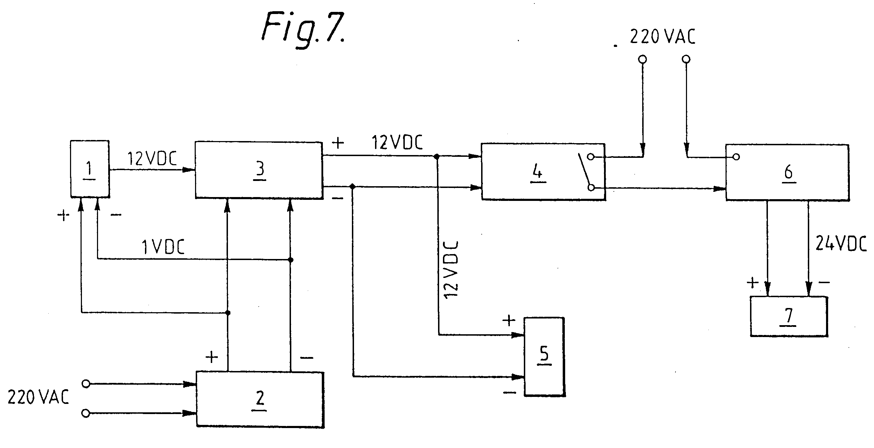

FIG. 7 shows a schematic diagram of the whole automatic circuit.

### DETAILED DESCRIPTION OF THE PREFERRED EMBODIMENT

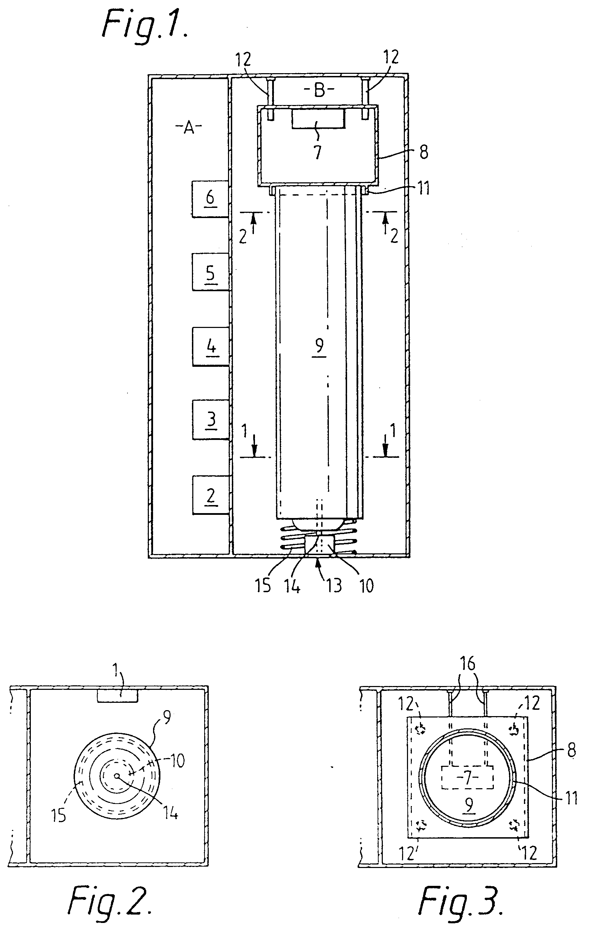

With reference to the drawing figures, a device according to this invention is shown in FIG.1. It comprises a housing consisting of 2 chambers,i.e. A and B. In chamber A, two power supplies **2** and **6**, a control circuit 3**,** a counter circuit **5,** and a solid state relay **4** are installed; while in chamber B a spray bottle **9** is placed in an upside-down manner with the spray nozzle **10** put through the opening **13**. The bottom of the spray bottle is held upright by an iron cap **11** fixed underneath the iron frame **8**. Four holes at each corner of the iron frame are pierced through by four plastic rods **12** which fixed to the roof of the device. The spring **15** at the bottom of chamber B also helps fixing the spray nozzle in place, where the plastic tubing **14** guides the fluid through the spray nozzle **10**. As shown in FIG. 1 and 3, the electromagnet **7** is fixed to the housing through the iron rods **16** with proper clearance from the inner side of the upper part of the iron frame **8**. Constructionally, the infrared light sensor **1** is passed through the floor of the device and located toward the back as shown in FIG. 2.

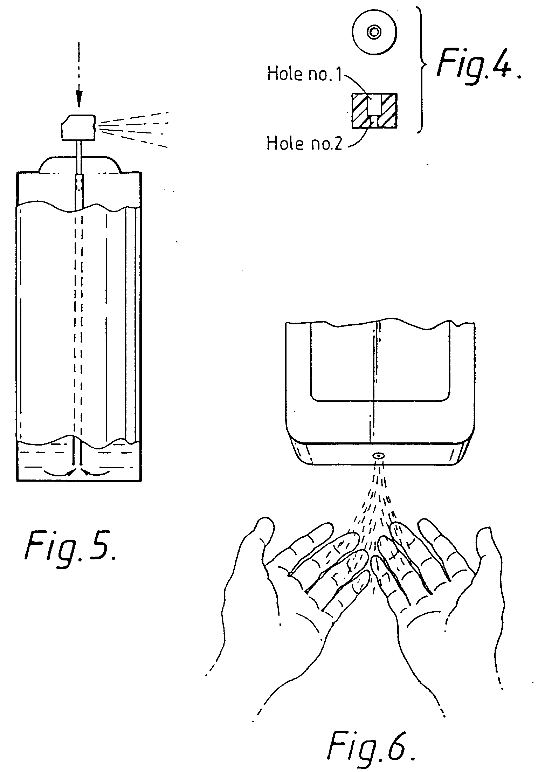

FIG. 5 illustrates the spray bottle operative in general, where the soft plastic tubing is used to guide the fluid through the spray nozzle under the pressure normally used for spraying. The spray bottle used in this device, however, does not need such soft plastic tubing. Only the hard short plastic tubing **14** to be fitted with hole no. 1, as shown in FIG. 4, of the spray nozzle is needed, while hole no. 2 of the spray nozzle has the diameter equal to that of the inner diameter of the hard plastic tubing **14**.

Upon introducing the hand palms underneath the device, approximately 20 cm. beneath the bottom of the device, as shown in FIG. 6, the infrared light sensor **1** will detect this and start the whole device. The control circuit **3** will then provide the solid state relay **4** the current of 12 VDC. The switch in the solid state relay **4** in turn will provide the power supply **6** with 220 VAC current such that the 24 VDC current can be provided to the electromagnet **7**. The electromagnet **7** will draw the iron frame **8** downward such that the bottom part of the iron frame **8** which fixed to the cap **11** will press onto the spray bottle in its upsidedown position. The valve in the spray nozzle within the bottle **10** will then be opened and the fluid disinfectant in the bottle will then be forced out of the spray nozzle **10** through the opening **13**. The volume of the fluid disinfectant to be forced out can be set that the optimal amount for maximal disinfection purpose could be achieved by adjusting the solid state relay **4**. Spraying of the disinfectant is discontinued immediately, and the next cycle can begin also immediately.

Rubbing the two hands together thoroughly will effectively disinfect the entire surface of both hand palms and the back of the hands.

The invention is designed such that when the fluid in the spray bottle is nearly used up, the counter circuit will start a warning sound to notify that a new full spray bottle of disinfectant should be installed and the counter circuit to be reset at 000 to start the machine a new round of operation.

This invention offers many important advantages over those prior devices.

The delivered liquid, in a finely nebulized form, is always spread over a broad area of the hand skin surface owing to the setting of their distance from the spray nozzle which is possible through adjusting of the infrared light. The hand palms are the first to be irrigated by simply introducing them up right beneath the device. Disinfection of skin is effectively complete by simply rubbing both hands together.

It should be noted that on no part of the device itself would the dispensed fluid be leftover so that it is almost unnesserary to clean the device regardingly. For this reason, contamination of the device is least possible which in turn would add up to the efficiency of the device for the disinfection purpose. In addition, the device as a whole is of simple construction which makes it easy to keep both inside and outside of it clean. As a whole, this device thus gives full assurance of being hygienic.

Moreover, the device of this invention has shown to be very quick to operate such that no time is needed by the next user to wait after the previous user finishes spraying, thus accordingly, may be used even where a large number of potential users is anticipated per unit, such as in hospitals, communities, and etc.

### Important aspects of the described invention are as follows:

The invention concerns the technical field of apparata for dispensing fluid over the hand palms for disinfection purpose, and relates to a spraying device for the dermatological treatment of the hands. The technical problem to be solved was that of providing a device which could spray fluids and readily irrigate in a fully automated manner the entire surface of the hands. The solution to the problem is provided by a device having a housing comprises of two chambers. In the first chamber(A), the power supplies, a control circuit, a counter circuit and a solid state relay are installed. In the second chamber(B), a spray bottle is installed in an upside-down manner with an electromagnet and an iron frame atop. At the bottom of the device, an infrared sensor(1) is arranged which responds to the presence of hands located in its direct proximity underneath. Upon activation, the fluid disinfectant is sprayed evenly on the hands to be irrigated to ensure efficient dermatological treatment where using of any hand drier devices is unnecessary.

## Claims

1. A fluid dispensing device for the dermatological treatment of hands, comprising:
a rigid housing, said rigid housing including a first chamber consisting of two sets of power supplies, a control circuit, a solid state relay, and a counter circuit;
a second chamber, said second chamber including a spray bottle of a dermatological disinfectant fluid, placed upside-down on a spray nozzle pointed downward with plastic tubing fitted into an opening at the bottom of said chamber held in place by a spring, and the bottom of said spray bottle held in upside-down position by an iron cap fixed to the bottom part of an iron frame. Inside said iron frame placed the electromagnet fixed to the housing through the iron rod with proper distance from the upper part of said iron frame;
and activating means within said chamber, comprising a proximity infrared light sensor in electrical communication with said control circuit and said solid state relay, for detecting the presence of hands at a predetermined position underneath said housing; said activating means, including means for causing said control circuit to operate supply the 12 VDC to said solid state relay, from where supplies a 220 VAC current into a second power supply which 24 VDC current is supplied to said electromagnet causing the drawing of said upper part of said iron frame onto the surface of said electromagnet; thus the lower part of said iron frame is pressed onto the bottom of said disinfectant spray bottle and the fluid is nebulized onto the hands when hands are present at said position; said counter circuit counts once for each operation cycle and turns on a sound switch to give a warning when the fluid in the spray bottle is nearly used up such that a new spray bottle should be installed.

2. The device of claim 1 wherein said first chamber lies adjacent to said second chamber; said proximity sensor is placed through the bottom of said second chamber and is calibrated to activate said control circuit to supply electric current to said electromagnetic system to operate when said hand is placed close to said proximity sensor.

3. The device of claim 2 wherein said electromagnetic system is activated to dispend a predeter mined volume of fluid.

4. Said control circuit within said device of claim 1 operates upon the activation of said proximity infrared sensor.
